# DEMANDE DE BREVET EUROPEEN

(11) **EP 1 190 697 A1**
(43) Date de publication de la demande: **27.03.2002**
(21) Numéro de dépôt: 01402395.6
(22) Date de dépôt: 19.09.2001
(51) Int. Cl.: A61K 7/075

(54) **Composition de lavage comprenant des particules d'oxyde d'aluminium, au moins un tensioactif anionique et au moins un tensioactif amphotère ou non ionique**

(30) Priorité: 20.09.2000 FR 0011994
(71) Demandeur: L'OREAL, 75008 Paris (FR)
(72) Inventeur: Perron, Béatrice, 78350 Jouy en Josas (FR); Restle, Serge, 95390 Saint Prix (FR); Giroud, Franck, 92110 Clichy (FR)
(74) Mandataire: Casalonga, Axel

(57) **Abrégé**

La présente invention concerne une composition de lavage des matières kératiniques qui comprend, dans un milieu aqueux cosmétiquement acceptable, des particules essentiellement constituées d'oxyde d'aluminium et de taille primaire moyenne en nombre inférieure à 200 nm, au moins un tensioactif anionique et au moins un tensioactif amphotère ou non ionique. L'invention concerne aussi un procédé de traitement cosmétique des fibres kératiniques, ainsi qu'une utilisation de la composition selon l'invention comme shampoing.

## Description

La présente invention est relative à une composition de lavage des matières kératiniques comprenant des particules essentiellement constituées d'oxyde d'aluminium, au moins un tensioactif anionique et au moins un tensioactif amphotère ou non ionique, à un procédé de traitement cosmétique des fibres kératiniques et à une utilisation de ladite composition comme shampoing.

Le brevet US 3 819 827 de WELLA décrit en particulier des produits pour la mise en plis des cheveux comprenant de 0,2 à 6 *%* en poids de particules d'oxyde d'aluminium présentant une taille de particule d'environ 30 mµ, et de 1 à 4 % en poids de polymères tels que, par exemple, la gomme adragante, l'agar-agar, la pectine, des polymères vinyliques et des polymères basiques.

La demanderesse a trouvé de manière surprenante que l'utilisation de particules essentiellement constituées d'oxyde d'aluminium et présentant une taille primaire moyenne en nombre inférieure à 200 nm, avec une base tensioactive particulière comprenant un tensioactif anionique et un tensioactif amphotère ou non ionique dans des compositions de lavage, permettait d'obtenir un bon maintien et un certain volume de la chevelure, c'est-à-dire un effet coiffant. On constate par ailleurs que les fibres kératiniques sont durcies et renforcées.

La présente invention a donc pour objet une composition de lavage comprenant dans un milieu aqueux cosmétiquement acceptable, des particules essentiellement constituées d'oxyde d'aluminium et présentant une taille primaire moyenne en nombre inférieure à 200 nm, au moins un tensioactif anionique et au moins un tensioactif amphotère ou non ionique.

Un autre objet de l'invention consiste en un procédé de traitement cosmétique des fibres kératiniques mettant en oeuvre la composition selon l'invention.

L'invention a encore pour objet une utilisation de la composition selon l'invention comme shampoing.

D'autres objets, caractéristiques, aspects et avantages de l'invention apparaîtront encore plus clairement à la lecture de la description et des divers exemples qui suivent.

Selon l'invention, la composition de lavage des matières kératiniques comprend, dans un milieu aqueux cosmétiquement acceptable, des particules essentiellement constituées d'oxyde d'aluminium et présentant une taille primaire moyenne en nombre inférieure à 200 nm, au moins un tensioactif anionique et au moins un tensioactif amphotère ou non ionique.

Par milieu aqueux cosmétiquement acceptable, on entend un milieu aqueux compatible avec les matières kératiniques telles que la peau et les cheveux.

Par "particules essentiellement constituées d'oxyde d'aluminium", on entend des particules constituées à plus de 90 % en poids d'oxyde d'aluminium.

Au sens de la présente invention, on entend par "taille primaire de particule", la dimension maximale qu'il est possible de mesurer entre deux points diamétralement opposés d'une particule individuelle. La taille peut être déterminée par microscopie électronique à transmission ou à partir de la mesure de la surface spécifique par la méthode BET.

La taille primaire moyenne en nombre des particules est de préférence comprise entre 5 et 50 nm.

Les particules d'oxyde d'aluminium selon l'invention sont essentiellement constituées par une quelconque alumine éventuellement hydratée telle que, par exemple, la boehmite.

Les particules peuvent présenter une forme quelconque telle que, par exemple, une forme de sphère, de paillettes, d'aiguilles ou de plaquettes. De préférence, elles sont sensiblement sphériques.

Les particules d'oxyde d'aluminium peuvent être utilisées dans la composition selon l'invention en une quantité allant de 0,01 à 20 % en poids, de préférence de 0,1 à 5 % en poids, par rapport au poids total de la composition de lavage de l'invention.

Comme tensioactifs anioniques utilisables dans la présente invention, on peut notamment mentionner les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkyl-amidoéthersulfates, les alkylarylpolyéthersulfates, les monoglycéride-sulfates ; les alkylsulfonates, les alkylamidesulfonates, les alkyl-arylsulfonates, les α-oléfine-sulfonates, les paraffine-sulfonates; les alkylsulfosuccinates, les alkyléthersulfosuccinates, les alkylamide-sulfosuccinates ; les alkylsulfoacétates ; les acylsarcosinates ; et les acylglutamates, les groupes alkyle et acyle de tous ces composés comportant de 6 à 24 atomes de carbone et le groupe aryle désignant de préférence un groupe phényle ou benzyle. On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Les tensioactifs anioniques tels que décrits ci-dessus, peuvent être utilisés seuls ou en mélange. On utilise, de préférence, les alkylsulfates, les alkyléthersulfates et les alkyléthercarboxylates, et leurs mélanges, en particulier sous forme de leurs sels de métaux alcalins ou alcalino-terreux, d'ammonium, d'amine ou d'aminoalcool.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL® , tels que décrits dans les brevets US-2 528 378 et US-2 781 354 et classés dans le dictionnaire CTFA, 3^{ème} édition, 1982, sous les dénominations Amphocarboxy-glycinate et Amphocarboxypropionate de structures respectives (1) et (2) :

Rₐ-CONHCH₂CH₂-N(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

R_{a'}-CONHCH₂CH₂-N(B)(C) (2)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
R_{a'} représente un groupe alkyle d'un acide R_{a'}-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylampho-dipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL® C2M concentré par la société RHODIA.

Les tensioactifs non-ioniques convenant dans l'invention sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)-aminopropylmorpholine; et leurs mélanges.

Parmi les tensioactifs non-ioniques cités ci-dessus, on utilise de préférence les alkyl(C₆-C₂₄)polyglycosides.

Les tensioactifs anioniques, les tensioactifs amphotères et les tensioactifs non-ioniques sont utilisés dans la composition de la présente invention en une quantité totale comprise entre 4 et 50 % en poids, de préférence entre 5 et 35 % en poids et mieux encore, entre 8 et 25 % en poids, par rapport au poids total de la composition.

La composition selon l'invention peut également comprendre en outre au moins un agent traitant soluble ou insoluble dans le milieu aqueux cosmétiquement acceptable.

Les agents traitants sont des composés bien connus et généralement utilisés dans la technique. A titre d'exemple, on peut notamment citer les saccharides, les oligosaccharides, les polysaccharides hydrolysés ou non, modifiés ou non ; les acides aminés ; les oligopeptides, les peptides ; les protéines hydrolysées ou non, modifiées ou non ; les acides et alcools gras ramifiés ou non ; les cires animales, végétales ou minérales ; les céramides et les pseudo-céramides ; les acides organiques hydroxylés ; les filtres UV ; les antioxydants et les agents anti-radicaux libres ; les chélatants ; les agents antipelliculaires ; les agents régulateurs de séborrée ; les agents apaisants ; les tensioactifs cationiques ; les polymères cationiques et amphotères ; les silicones organomodifiées ou non ; les huiles minérales, végétales, animales ou synthétiques ; les polyisobutènes et poly(α-oléfines) ; les esters ; les polymères anioniques solubles ou dispersés ; les polymères non-ioniques solubles ou dispersés ; et leurs mélanges.

Ces agents traitants sont utilisés dans la composition en une quantité efficace, c'est-à-dire en une quantité permettant d'obtenir les effets traitants recherchés par l'homme de l'art.

Le milieu aqueux cosmétiquement acceptable peut être constitué uniquement par de l'eau ou par un mélange d'eau et d'un solvant cosmétiquement acceptable tel qu'un alcool inférieur en C₁-C₄, tel que l'éthanol, l'isopropanol, le tertio-butanol, le n-butanol ; les alkylèneglycols comme le propylèneglycol, les éthers de polyol ; les alcanes en C₅-C₁₀ ; l'acétone, la méthyléthylcétone ; les acétates d'alkyle en C₁-C₄ comme l'acétate de méthyle, l'acétate d'éthyle, l'acétate de butyle ; le diméthoxyéthane, le diéthoxyéthane ; et leurs mélanges.

Le pH des compositions de l'invention est compris entre 4 et 8, de préférence entre 5 et 7.

Les compositions selon l'invention peuvent également contenir en outre des additifs tels que des épaississants polymériques naturels ou synthétiques, anioniques, amphotères, zwittérioniques, non-ioniques ou cationiques, associatifs ou non, des épaississants non polymériques comme des acides ou des électrolytes, des nacrants, des opacifiants, des solvants organiques, des parfums, des colorants, des particules organiques, des conservateurs, des agents de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions peuvent se présenter sous forme de liquides fluides ou épaissis, de gels, de crèmes, de mousses, d'émulsions eau-dans l'huile (E/H), huile-dans-eau (H/E) ou d'émulsions multiples.

Elles peuvent être utilisées, par exemple, comme shampoings, soins rincés, masques de soin profond, lotions ou crèmes de traitement du cuir chevelu.

La présente invention concerne également un procédé de traitement cosmétique qui consiste à appliquer une quantité efficace d'une composition telle que décrite ci-dessus, sur les fibres kératiniques, à effectuer un rinçage après un éventuel temps de pose.

Selon un mode de réalisation préféré de l'invention, la composition peut être utilisée comme shampoing.

Les exemples suivants illustrent la présente invention et ne doivent être considérés en aucune manière comme limitant l'invention.

### EXEMPLES

On a préparé les shampoings de compositions A et B suivantes à partir des ingrédients indiqués dans le tableau ci-dessous. Les quantités sont indiquées en % en poids par rapport au poids total de la composition.

| Composition | A | B |
|---|---|---|
| Oxyde d'aluminium⁽¹⁾ | 0,5 | 0,5 |
| Lauryléthersulfate de sodium (2,2 moles d'oxyde d'éthylène) à 26 % de matière active | 47,5 | 47,5 |
| Cocoamphodiacétate de sodium à 38 % de matière active | 6,6 | 6,6 |
| JR400 (AMERCHOL) | - | 0,5 |
| Eau | qsp 100 | qsp 100 |
| pH | 6,5 | 6,5 |

| | | |
|---|---|---|
| ⁽¹⁾ présentant une taille de particule primaire moyenne en nombre de 13 nm, vendu sous la dénomination ALUMINIUMOXID C par la société DEGUSSA-HULS. | | |

On applique les compositions A et B selon l'invention sur les cheveux, on rince et on fait sécher les cheveux.

On observe un renforcement des fibres kératiniques et les cheveux se coiffent mieux.

## Revendications

1. Composition de lavage des matières kératiniques, **caractérisée en ce qu'**elle comprend, dans un milieu aqueux cosmétiquement acceptable, des particules essentiellement constituées d'oxyde d'aluminium de taille primaire moyenne en nombre inférieure à 200 nm, au moins un tensioactif anionique et au moins un tensioactif amphotère ou non ionique, et **en ce que** la quantité totale de tensioactifs est comprise entre 4 et 50 % en poids par rapport au poids total de la composition.

2. Composition de lavage selon la revendication 1, **caractérisée en ce que** la taille primaire moyenne en nombre des particules est comprise entre 5 et 50 nm.

3. Composition de lavage selon la revendication 1 ou 2, **caractérisée en ce que** l'oxyde d'aluminium est une alumine éventuellement hydratée.

4. Composition de lavage selon la revendication 3, **caractérisée en ce que** l'oxyde d'aluminium est la boehmite.

5. Composition de lavage selon l'une quelconque des revendications précédentes, **caractérisée en ce que** les particules sont présentes en une concentration de 0,01 à 20 % en poids par rapport au poids total de la composition.

6. Composition de lavage selon la revendication 5, **caractérisée en ce que** les particules sont présentes en une concentration de 0,1 à 5 % en poids par rapport au poids total de la composition.

7. Composition de lavage selon l'une quelconque des revendications précédentes, **caractérisée en ce que** les tensioactifs anioniques sont choisis parmi les sels des types suivants : les alkylsulfates, les alkyléthersulfates, les alkylamidoéthersulfates, les alkylarylpolyéthersulfates, les monoglycéride-sulfates ; les alkyl-sulfonates, les alkylamidesulfonates, les alkylarylsulfonates, les α-oléfine-sulfonates, les paraffine-sulfonates ; les alkylsulfosuccinates, les alkyléthersulfosuccinates, les alkylamidesulfosuccinates ; les alkylsulfoacétates ; les acylsarcosinates ; et les acylglutamates, les groupes alkyle ou acyle de ces composés comportant de 6 à 24 atomes de carbone et le groupe aryle désignant un groupe phényle ou benzyle ; les esters d'alkyle en C₆-C₂₄ et d'acides polyglycoside-carboxyliques ; les alkylsulfosuccinamates, les acyliséthionates et les N-acyltaurates, le groupe alkyle ou acyle comportant de 12 à 20 atomes de carbone ; les acyllactylates dont le groupe acyle comporte de 8 à 20 atomes de carbone ; les acides d'alkyl-D-galactoside uroniques et leurs sels, les acides alkyl(C₆-C₂₄)éther-carboxyliques polyoxyalkylénés, les acides alkyl(C₆-C₂₄)-aryl(C₆-C₂₄)éther-carboxyliques polyoxyalkylénés, les acides alkyl(C₆-C₂₄)amidoéther-carboxyliques polyoxyalkylénés et leurs sels ; et leurs mélanges.

8. Composition de lavage selon la revendication 7, **caractérisée en ce que** les tensioactifs anioniques sont choisis parmi les alkylsulfates, les alkyléthersulfates et les alkyléthercarboxylates, et leurs mélanges, sous forme de sels de métaux alcalins ou alcalino-terreux, d'amine, d'ammonium ou d'aminoalcool.

9. Composition de lavage selon l'une quelconque des revendications précédentes, **caractérisée en ce que** les tensioactifs amphotères sont choisis parmi des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique hydrosolubilisant ; les alkyl(C₈-C₂₀)-bétaïnes, les sulfobétaïnes, les alkyl(C₈-C₂₀)amidoalkyl(C₆-C₈)-bétaïnes ou les alkyl(C₈-C₂₀)amidoalkyl (C₆-C₈)sulfobétaïnes ; et leurs mélanges.

10. Composition de lavage selon l'une quelconque des revendications précédentes, **caractérisée en ce que** les tensioactifs non-ioniques sont choisis parmi les alcools, les alpha-diols, les alkyl(C₁-C₂₀)phénols ou les acides gras en C₈-C₁₈ polyéthoxylés, polypropoxylés ou polyglycérolés, le nombre de groupements oxyde d'éthylène ou oxyde de propylène allant de 2 à 50 et le nombre de groupements glycérol allant de 2 à 30, les copolymères d'oxyde d'éthylène et de propylène, les condensats d'oxyde d'éthylène et de propylène sur des alcools gras ; les amides gras polyéthoxylés ayant de 2 à 30 moles d'oxyde d'éthylène, les amides gras polyglycérolés comportant en moyenne 1 à 5 groupements glycérol ; les amines grasses polyéthoxylées ayant de 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines ; et leurs mélanges.

11. Composition de lavage selon l'une quelconque des revendications précédentes, **caractérisée en ce que** les tensioactifs sont utilisés en une quantité comprise entre 8 et 25 % en poids, par rapport au poids total de la composition.

12. Composition de lavage selon l'une quelconque des revendications précédentes, **caractérisée en ce qu'**elle comprend en outre au moins un agent traitant soluble ou insoluble dans le milieu aqueux cosmétiquement acceptable.

13. Composition de lavage selon la revendication 12, **caractérisée en ce que** l'agent traitant est choisi parmi les saccharides, les oligosaccharides, les polysaccharides hydrolysés ou non, modifiés ou non ; les acides aminés ; les oligopeptides, les peptides ; les protéines hydrolysées ou non, modifiées ou non ; les acides et alcools gras ramifiés ou non ; les cires animales, végétales ou minérales ; les céramides et les pseudo-céramides ; les acides organiques hydroxylés ; les filtres UV ; les antioxydants et les agents anti-radicaux libres ; les chélatants ; les agents antipelliculaires ; les agents régulateurs de séborrée ; les agents apaisants ; les tensioactifs cationiques ; les polymères cationiques et amphotères ; les silicones organomodifiées ou non ; les huiles minérales, végétales, animales ou synthétiques ; les polyisobutènes et poly(α-oléfines) ; les esters ; les polymères anioniques solubles ou dispersés ; les polymères non-ioniques solubles ou dispersés ; et leurs mélanges.

14. Composition de lavage selon la revendication 13, **caractérisée par le fait que** l'agent traitant est un polymère cationique ou amphotère.

15. Composition de lavage selon l'une quelconque des revendications précédentes, **caractérisée en ce que** le milieu aqueux cosmétiquement acceptable est constitué uniquement par de l'eau ou par un mélange d'eau et d'un solvant cosmétiquement acceptable.

16. Composition de lavage selon la revendication 15, **caractérisée en ce que** le solvant cosmétiquement acceptable est choisi parmi les alcools inférieurs en C₁-C₄, les alkylèneglycols, les alcanes en C₅-C₁₀, l'acétone, la méthyléthylcétone, les acétates d'alkyle en C₁-C₄, le diméthoxyéthane , le diéthoxyéthane et leurs mélanges.

17. Composition de lavage selon l'une quelconque des revendications précédentes, **caractérisée en ce qu'**elle comprend en outre des additifs tels que des épaississants polymériques naturels ou synthétiques, anioniques, amphotères, zwitterioniques, non-ioniques ou cationiques, associatifs ou non, des épaississants non polymériques comme des acides ou des électrolytes, des nacrants, des opacifiants, des parfums, des colorants, des particules organiques, des conservateurs, des agents de stabilisation du pH.

18. Procédé de traitement cosmétique des fibres kératiniques, **caractérisé en ce que** l'on applique sur les fibres kératiniques une composition de lavage selon l'une quelconque des revendications précédentes, et que l'on effectue un rinçage après un éventuel temps de pose.

19. Utilisation d'une composition de lavage selon l'une quelconque des revendications 1 à 17, comme shampoing.
